# EUROPEAN PATENT SPECIFICATION

(11) **EP 3 841 077 B1**
(45) Date of publication and mention of the grant of the patent: **13.04.2022**
(21) Application number: 19745189.1
(22) Date of filing: 11.07.2019
(51) Int. Cl.: C05F 17/90, C05F 17/964

(54) **AN APPARATUS AND A METHOD FOR COMPOSTING ORGANIC MATERIAL**
VORRICHTUNG UND VERFAHREN ZUM KOMPOSTIEREN VON ORGANISCHEM MATERIAL
APPAREIL ET PROCÉDÉ DE COMPOSTAGE DE MATÉRIAU ORGANIQUE

(30) Priority: 24.08.2018 FI 20185697
(43) Date of publication of application: 30.06.2021
(73) Proprietor: Biopallo Systems Oy, 70460 Kuopio (FI)
(72) Inventor: SUHONEN, Anssi, 70460 Kuopio (FI)
(74) Representative: Kolster Oy Ab
(86) International application number: PCT/FI2019/050543
(87) International publication number: WO 2020/039118

(56) References cited:
- EP-A1- 1 387 817
- DE-A1- 3 844 700
- DATABASE EPODOC [Online] EUROPEAN PATENT OFFICE, THE HAGUE, NL; 3 March 2011 (2011-03-03), PARK YOUNG HYUN [KR]; KIM JUNG LEE [KR]: "CRUSHER FOR LEAF ABSCISSION", XP002794188, Database accession no. KR-20100119705-A & KR 101 019 144 B1 (KIM JUNG LEE [KR]; PARK YOUNG HYUN [KR]) 3 March 2011 (2011-03-03)
- DATABASE WPI Week 201683 Thomson Scientific, London, GB; AN 2016-76785V XP002794189, & KR 2016 0136896 A (JEON Y G) 30 November 2016 (2016-11-30)
- DATABASE WPI Week 200027 Thomson Scientific, London, GB; AN 2000-306503 XP002794190, & JP 3 029427 B1 (UEDA H) 4 April 2000 (2000-04-04)

## Description

### FIELD

The invention relates to an apparatus and a method for composting organic material as described in claims 1 and 13 respectively.

### BACKGROUND

There are various prior art methods for composting organic material, such as biowaste and biomass and various devices used in the methods. Such devices are, for example, tunnel composters, drum composters and silo composters. The material to be composted in these composters is fed to the composter after which the conditions in the composters are regulated so that the composting process begins and proceeds in a functional way. Air may be fed to the composter during the composting process in order to supply oxygen into the composting process.

The prior art composting methods require a long time, e.g. usually several weeks, for the organic material to compost and the control of the composting process is difficult in the prior art methods. Another problem in prior art methods is that the organic material composts heterogeneously. It is also difficult to verify the proceeding of the stages of the process and the hygiene of the process in prior art composting methods. The control of the odour is also not efficient in current composting methods. The workers may even be exposed to health hazards in prior art composters.

DE 3844700 discloses a composting device comprising a container with a lower portion and an upper portion. The organic material is introduced from the side to the lower portion of the container to a cutting and mixing device. The material is further transported upwards with a screw. The outlet opening is at the upper end of the upper portion of the container.

EP 1 387 817 discloses a method and an apparatus for composting organic material. The apparatus comprises a container having a first end and a second end formed as a hemispherical wall. A transport and mixing device transports organic material from the first end to the second end of the container.

KR101019144 B ,KR20160136896 A or JP2000247768 A also disclose grinding devices for organic matter.

### SUMMARY

An object of the present invention is to present an improved apparatus and method for composting organic material.

The apparatus for composting organic material according to the invention is defined in claim 1.

The method for composting organic material according to the invention is defined in claim 13.

The apparatus for composting organic material comprises
a container having a first end, a second opposite end, and a jacket extending between the first end and the second end, the second end being formed as a hemispherical wall, the container comprising at least one inlet opening for the organic material and at least one outlet opening for composted material, the container operating in an inclined position in which the second end of the container is on a higher horizontal level than the first end of the container,
a transporting and mixing device positioned in the container for transporting organic material in a transport direction from the first end to the second end of the container and simultaneously mixing the organic material, the transporting and mixing device directing the organic material towards the hemispheric wall, whereby the organic material is first directed upwards along the hemispheric wall and then turned back against the transport direction.

The apparatus is characterized in that
a fluffing device is positioned in the container for cutting the organic material into a smaller particle size and for throwing at least a portion of the organic material backwards towards the first end of the container, the fluffing device comprising a shaft extending substantially perpendicularly in relation to the transport direction and cutter blades extending substantially perpendicularly outwards from the shaft.

The method for composting organic material comprises
providing organic material from at least one inlet opening to a container having a first end and an opposite second end formed as a hemispheric wall, the container operating in an inclined position in which the second end of the container is on a higher horizontal level than a first end of the container,
providing additional material for regulating the oxygen content and/or bacteria and/or enzymes to the container in order to agitate the composting process in the container,
transporting the organic material with a transporting and mixing device in a transport direction from the first end towards the hemispheric wall at the second end of the container, whereby the organic material is first directed upwards along the hemispheric wall and then turned back towards the first end of the container, the organic material also being mixed during the transport,
removing the composted material from at least one outlet opening in the container after the composting process within the container has been completed,

The method is characterised in
cutting the organic material into a smaller particle size and throwing at least a portion of the organic material backwards towards the first end of the container with a fluffing device comprising a shaft extending substantially perpendicularly in relation to the transport direction and cutter blades extending outwards from the shaft.

The apparatus for composting organic material provides a fast, homogeneous and flexible composting process within the container. It is easy to provide suitable measurements within the container in order to be able to verify how the composting process proceeds. The apparatus for composting organic material is also simple and reliable.

The time to compost a batch of organic material in the apparatus for composting organic material is only 1 to 3 days. The composted material may then be removed from the at least one outlet opening to a site outside the container in order to be stabilized.

The fluffing device will intensify the cutting and/or chopping of the organic material into a smaller particle size within the container. The smaller particle size will contribute to a more efficient oxidation of the organic material within the container.

The fluffing device will also throw at least a portion of the organic material backwards towards the first end of the container in order to achieve an efficient rotation and mixing of the organic material within the container.

The fluffing device will also contribute to eliminate the vaulting of the organic material on the walls of the container at the second end of the container. This vaulting may be a problem especially when the moisture content of the organic material is high within the container.

At least the moisture content and the temperature within the container may advantageously be measured by a suitable moisture sensor and temperature sensor positioned within the container. The moisture content within the container may be regulated by providing fresh air to the container. The moisture content of the air that is supplied into the container may be regulated, whereby also the moisture content within the container becomes adjusted. The supply of fresh air into the container will naturally also provide oxygen into the container.

The pH-value of the organic material within the container may further be measured. Acidity regulating material may then be added to the container in order to adjust the acidity of the organic material within the container. The acidity of the organic material has an influence on the functioning of the bacteria de-composting the organic material within the container. By adding lime or some other acidity controlling material into the container, it is possible to keep the pH-value of the organic material within the container at a suitable level for an efficient composting process.

The optimal mixing and/or oxidation of the organic material within the container may be regulated by regulating the inclination angle of the container and/or by regulating the rotation speed of the screws in the transporting and mixing device and/or by regulating the rotation speed of the fluffing device. The air circulation within the container and/or the removal of moisture from the container may further be regulated by regulating the supply of air to the container.

### DRAWINGS

The invention will in the following be described in greater detail by means of preferred embodiments with reference to the attached drawings, in which:
Figure 1 shows a side view of an apparatus for composting organic material,
Figure 2 shows a side view of the transporting and mixing device,
Figure 3 shows an upper view of the transporting and mixing device and the fluffing device,
Figure 4 shows an enlarged upper view of the transporting and mixing device and the fluffing device,
Figure 5 shows an axonometric view of the transporting and mixing device and the fluffing device,
Figure 6 shows an axonometric view of the fluffing device,
Figure 7 shows an axonometric view of a blade in the fluffing device.

### DETAILED DESCRIPTION

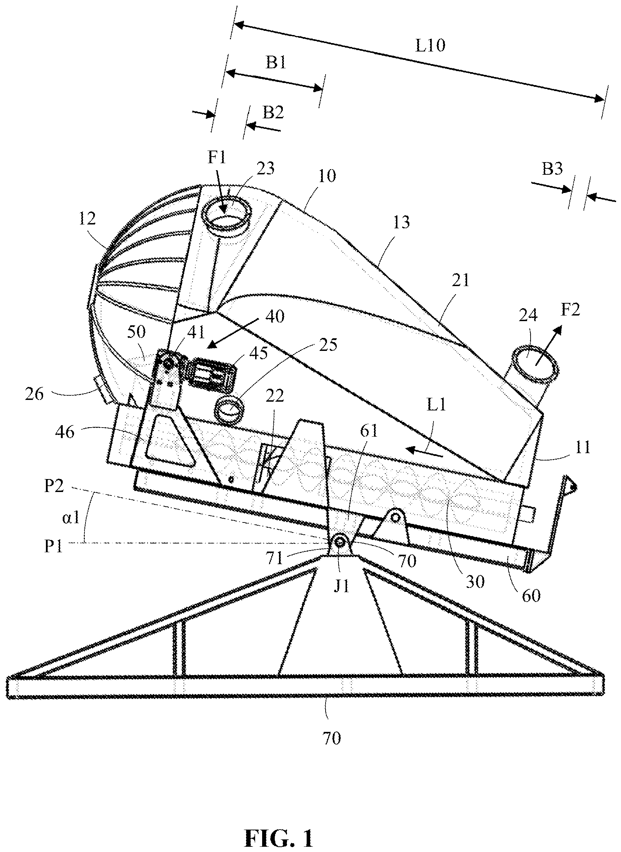

Figure 1 shows a side view of an apparatus for composting organic material.

The apparatus for composting organic material comprises a container 10, a transporting and mixing device 30, and a fluffing device 40.

The container 10, the transporting and mixing device 30, and a fluffing device 40 are supported on a first frame structure 60. The first frame structure 60 is positioned below the transporting and mixing device 30.

The container 10 has a first end 11, a second opposite end 12, and a jacket 13 extending between the first end 11 and the second end 12. The second end 12 of the container 10 may be formed as a hemispherical wall. The jacket 13 of the container 10 may have a substantially conical form so that the cross section of the jacket 13 contracts from the beginning of the hemispherical wall at the second end 12 of the container 10 towards the first end 11 of the container 10. The first end 11 of the container may be inclined.

The container 10 in this embodiment has at least one inlet opening 21 for the organic material and at least one outlet opening 22 for composted material. The container 10 in this embodiment has further at least a first air opening 23 through which air can be directed into the interior of the container 10 and at least a second air opening 24 through which air can be directed out from the interior of the container 10. Fresh air and thereby oxygen can be provided to the interior of the container 10 from the first air opening 23. The supply of oxygen to the container 10 will contribute to the speed and to the control of the composting process. The container 10 in this embodiment has further measuring pockets 25, 26 for measuring the temperature within the container 10.

The container 10 may in an embodiment be provided with one first air opening 23 and one second air opening 24.

The container 10 may be operated in an inclined α1 position in which the second end 12 of the container 10 may be on a higher horizontal level than the first end 11 of the container 10.

The transporting and mixing device 30 is positioned in the container 10. The transporting and mixing device 30 may be positioned substantially at a bottom of the container 10. The transporting and mixing device 30 may transport organic material in a transport direction L1 from the first end 11 to the second end 12 of the container 10. The transporting and mixing device 30 may mix the organic material simultaneously during the transport of the organic material from the first end 11 to the second end 12 of the container 10. The transporting and mixing device 30 may direct the organic material towards the hemispheric wall at the second end 12 of the container 10. The organic material is first directed upwards along the hemispheric wall at the second end 12 of the container 10. The organic material is after that turned back against the transport direction L1 of the organic material in the upper portion of the hemispherical wall. The organic material will fall down from the upper portion of the hemispherical wall and the upper portion of the jacket 13 of the container 10 on the transporting and mixing device 30 in order to be transported again in the transport direction L1 against the second end 12 of the container 10.

The fluffing device 40 is positioned in the container 10. The fluffing device 40 may comprise a shaft 41 extending substantially perpendicularly in relation to the transport direction L1. The shaft 41 may be provided with cutter blades 50 extending outwards from the shaft 41. The cutter blades 50 may extend perpendicularly outwards from the shaft 41. The cutter blades 50 may be attached to the shaft 41 so that rotation of the shaft 41 will also rotate the cutter blades 50. The fluffing device 40 may be driven by an electric motor 45. The electric motor 45 may be connected to the shaft 41 of the fluffing device 40. The fluffing device 40 is used to cut and fluff the organic material. The shaft 41 of the fluffing device 40 may be positioned substantially at the beginning of the spherical wall at the second end 12 of the container 10.

The position of the shaft 41 of the fluffing device 40 shown in the figure i.e. substantially at the beginning of the spherical wall at the second end 12 of the container 10 is an advantageous position. The fluffing device 40 may in this position cut and fluff the organic material just before it is entering the hemispheric wall at the second end 12 of the container 10. The cutter blades 50 of the fluffing device 40 may be formed so that a portion of the organic material is thrown back towards the first end 11 of the container 10 by the cutter blades 50 when they rotate with the shaft 41 of the fluffing device 40.

The beginning of the hemispherical wall is the junction between the jacket 13 and the hemispherical wall at the second end 12 of the container 10. The beginning of the hemispheric wall is thus the point at which a diameter of a cross section of the hemispherical wall reaches the greatest value.

The shaft 41 of the fluffing device 40 may be positioned at a first distance B1 from the beginning of the hemispherical wall at the second end 12 of the container 10. This first distance B1 may be in the range of 0-20%, preferably in the range of 0-10% of a first length L10 extending from the first end 11 of the container 10 to the beginning of the hemispheric wall at the send end 12 of the container 10. The first distance B1 and the first length L10 may extend in the transport direction L1.

The first air opening 23 may be positioned in an upper portion of the container 10. The first air opening 23 may be positioned substantially above the fluffing device 40. The first air opening 23 may be positioned at a second distance B2 from the beginning of the hemispherical wall at the second end 12 of the container 10. This second distance B2 may be in the range of 0-20%, preferably in the range of 0-10% of the first length L10 extending from the first end 11 of the container 10 to the beginning of the hemispheric wall at the send end 12 of the container 10. The second distance B2 may also extend in the transport direction L1.

A flow of fresh inlet air F1 may thus be directed towards the flow of organic material thrown backwards in the container with the fluffing device 40. The rotational speed of the fluffing device 40 may be regulated so that the portion of organic material which is thrown backwards towards the first end 11 of the container 10 with the fluffing device 40 is transferred into a mist containing droplets of organic material. The fresh inlet air F1 blown into the container 10 will thus be directed towards the mist of organic material, whereby an efficient oxidation of the organic material is achieved.

The second air opening 24 may be positioned in an upper portion of the container 10. The second air opening 24 may be positioned above the transporting and mixing device 30. The second air opening 24 may be positioned at a third distance B3 from the first end 11 of the container 10. This third distance B3 may be in the range of 0-20%, preferably in the range of 0-10% of the first length L10 extending from the first end 11 of the container 10 to the beginning of the hemispherical wall at the second end 12 of the container 10. The third distance B3 may also extend in the transport direction L1.

A continuous air circulation may be provided through the container 10 so that air F1 is directed into the container 10 from the first air opening 23 and air F2 is directed out from the container 10 from the second air opening 24. A blower positioned outside the container 10 may be used to produce the air circulation through the container 10.

The first air opening 23 and the second air opening 24 may be positioned in an upper portion of the jacket 13 of the container 10. The first air opening 23 and the second air opening 24 may thus be positioned above the transporting and mixing device 40. The first air opening 23 may further be positioned substantially above the fluffing device 40.

The container 10 may be adjustable between a horizontal first position P1 and an inclined α1 second position P2 in which the second end 12 of the container 10 is on a higher level than the first end 11 of the container 10.

The adjustability of the container 10 between the first and the second position P1, P2 may be achieved by supporting the container 10 pivotably J1 on a second frame structure 70. The container 10 may be arranged to be turnable around the pivot point J1 between the horizontal first position P1 and the inclined α1 second position P2. The pivot J1 support may be arranged between the first frame structure 60 and the second frame structure 70. The first frame structure 60 may comprise downwardly directed first lugs 61 at each side of the first frame structure 60. The second frame structure 70 may comprise corresponding upwardly directed second lugs 71 at each side of the second frame structure 70. The first and the second lugs 61, 71 may comprise a hole so that a shaft 80 may pass through the holes in the first and the second lugs 61, 71. The first frame structure 60 and thereby also the container 10 is thus pivotable around the shaft 80.

The turning of the container 10 between the first position P1 and the second position P2 and vice a versa may be done with any suitable actuators e.g. with hydraulic cylinders or electric motors connected to cogwheels. (This is not for clarity reasons shown in the figures).

The container 10 may be operated in batch runs. The container 10 may first be positioned in the horizontal first position P1 and filled with organic material through the inlet opening 21 to approximately one third of the volume of the container 10. The container 10 may thereafter be turned into the inclined second position P2 in which second position P2 the container 10 is operated during the composting process. The transporting and mixing device 30 may then be operated in desired intervals during the composting process. The fluffing device 40 may also be operated in desired intervals during the composting process. The composting conditions within the container 10 may be measured continuously or at certain intervals during the composting process. The transporting and mixing device 30 as well as the fluffing device 40 may be operated based on the composting conditions within the container 10 determined from the results of the measurements.

The transporting and mixing device 30 and the fluffing device 40 may each have a driving motor of their own. This would mean that the two devices 30, 40 may be operated so that the operation intervals of the two devices 30, 40 coincide fully or partly or so that there is no overlapping of the operation intervals of the two devices 30, 40. Also the rotational speed of each device 30, 40 could then be regulated independently. The transporting and mixing device 30 and the fluffing device 40 may on the other hand be operated by a common driving motor. One of the two devices 30, 40 could be connected directly to the driving motor and the other device 30, 40 could be connected through a transmission to the driving motor or both devices 30, 40 could be connected through a transmission to the driving motor. This would mean that the two devices 30, 40 may be operated only in synchronism with each other. The rotational speed of the devices 30, 40 could be different, but the ratio between the rotational speeds of the devices 30, 40 would be constant. This ratio could be determined by the transmission.

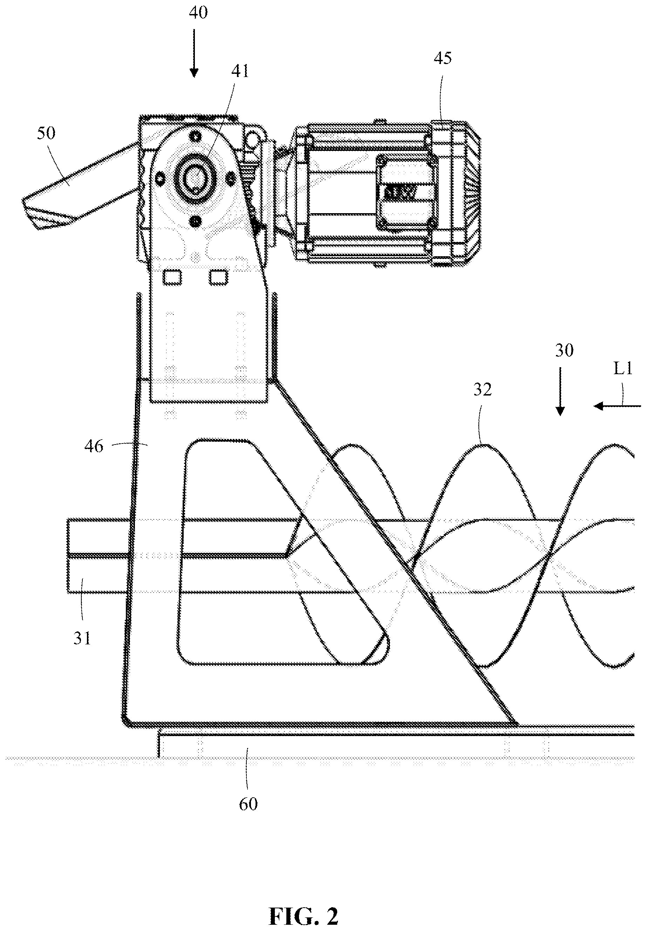

Figure 2 shows a side view of the transporting and mixing device.

The transporting and mixing device 30 may comprise a cylindrical shaft 31 provided with a screw 32 extending outwards from the outer perimeter of the shaft 31. Rotation of the shaft 31 rotates the screw 32 whereby material is transported with the screw 32 from the first end 11 of the container 10 to the second end 12 of the container 10. The transporting and mixing device 30 may be driven by an electric motor (not shown in the figure) connected to the shaft 31 of the transporting and mixing device 30. The threading of the screw 32 transports the material in the axial direction of the transporting and mixing device 30 when the shaft 31 is rotated.

The fluffing device 40 may comprise a shaft 41 extending in a direction which is substantially perpendicular in relation to the transport direction L1. The shaft 41 may be positioned within the container 10 substantially at the beginning of the spherical wall at the second end 12 of the container 10. The fluffing device 40 may further comprise cutter blades 50 extending perpendicularly outwards from the shaft 41.

The fluffing device 40 may be driven by an electric motor 45 connected to the shaft 41 of the fluffing device 40.

Both ends of the shaft 41 of the fluffing device 40 may be rotatably supported e.g. through bearings on a support structure 46. The support structure 46 may be supported on the first frame construction 50. The fluffing device 40 may be positioned above the transporting and mixing device 30 in the vertical direction. The cutter blades 50 of the fluffing device 40 may thus rotate freely without interfering with the screw 32 of the transporting and mixing device 30.

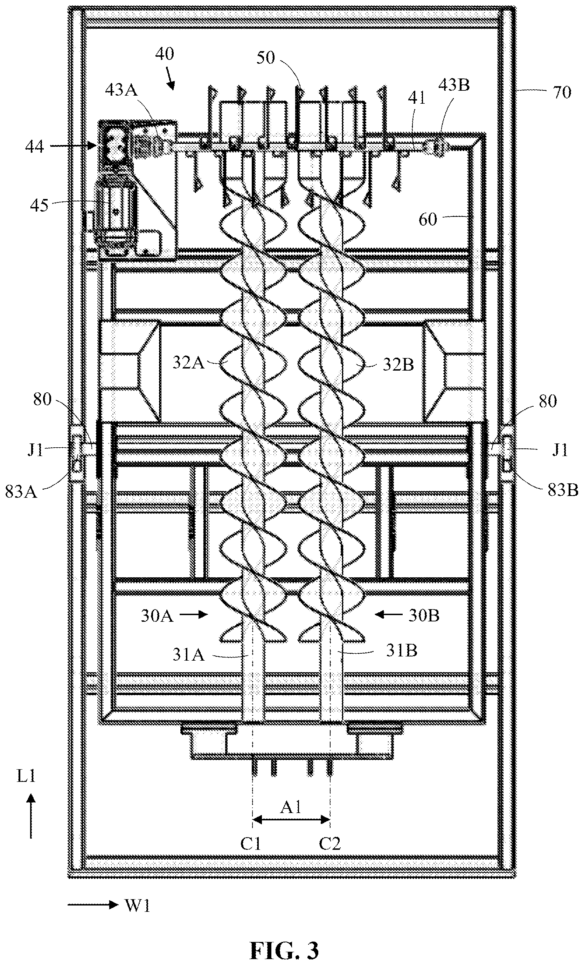

Figure 3 shows an upper view of the mixing and transport device and the fluffing device.

This transporting and mixing device 30 in this embodiment comprises two longitudinal in the length direction L1 of the apparatus directed screw means 30A, 30B. The length direction L1 of the apparatus coincides with the transport direction L1 of the apparatus. Each screw means 30A, 30B comprises a shaft 31A, 31B and a screw 32A, 32B extending outwards from the perimeter of the shaft 31A, 31B. The longitudinal centre axes C1, C2 of the shafts 31A, 31B of the two screw means 30A, 30B are parallel and positioned at a distance A1 from each other. The tips of the screws 32A, 32B of the screw means 30A, 30B are positioned at a distance from each other so that the screws 32A, 32B can rotated without contacting each other and without disturbing each other.

The screws 32A, 32B are open i.e. there is no pipe surrounding the screws 32A, 32B. The threads of the screws 32A, 32B in both screw means 30A, 30B are directed so that the material is transported with the screw 32A, 32B from the first end 11 to the second end 12 of the container 10. The threads of the screws 32A, 32B may be identical.

The shafts 31A, 31B of the screw means 30A, 30B may be supported via bearings to the first frame construction 60 (this is not for clarity reasons shown in the figures).

One end of the shafts 31A, 31B of the screw means 30A, 30B may be connected to a drive motor (this is not for clarity reasons shown in the figures). The two shafts 31A, 31B may be driven by a common drive motor connected through a transmission to the two shafts 31A, 31B. The other possibility is that each of the shafts 31A, 31B is driven by a drive motor of their own. The drive motor may be an electric motor.

Each end of the shaft 41 of the fluffing device 40 is rotatably supported via a bearing 43A, 43B on the first frame structure 60. A first end of the shaft 41 of the fluffing device 40 may be connected via a transmission 44 to the drive motor 45. The rotational speed of the shaft 41 of the fluffing device 40 may be the same or it may be different i.e. greater or smaller than the rotational speed of the shaft of the drive motor 45. The ratio between the rotational speeds of the fluffing device 40 and the drive motor 45 may be determined by the transmission.

The first frame structure 60 may be supported via a shaft 80 on the second frame construction 70. The shaft 80 may extend in the width direction W1 of the apparatus over the whole width of the first frame structure 60 or the shaft 80 may be formed of two pins extending outwards from the sides of the first frame construction 60. The end of the shaft 80 may be supported with bearings 83A, 83B on the second frame construction 70. The shaft 80 forms a pivot point J1 between the first frame construction 60 and the second frame construction 70. The first frame construction 60 may be turned in relation to the second frame construction 70 around the pivot point J1.

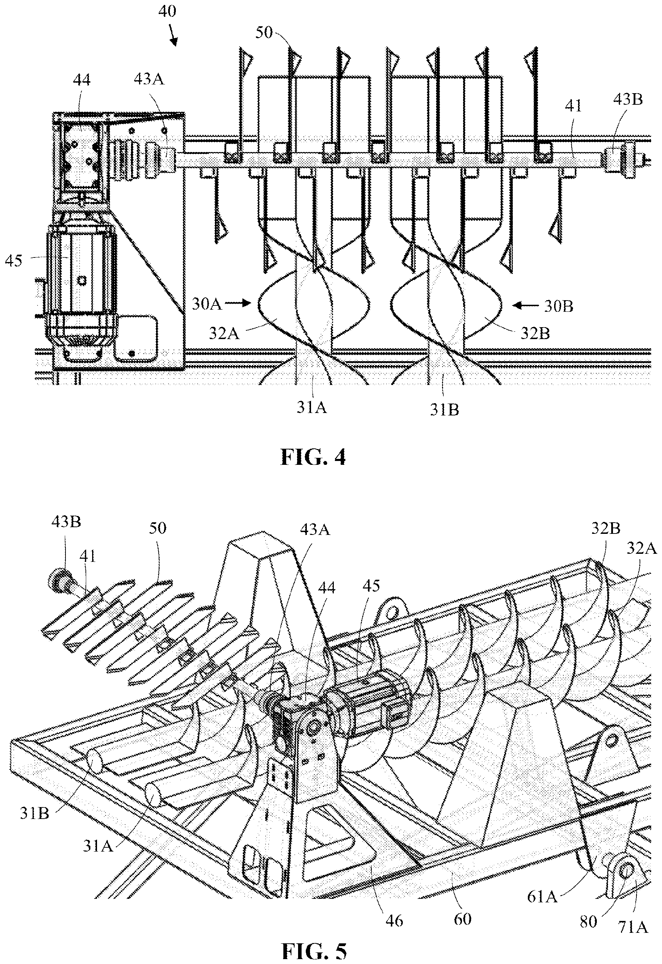

Figure 4 shows an enlarged upper view of the transporting and mixing device and the fluffing device and figure 5 shows an axonometric view of the transporting and mixing device and the fluffing device.

The fluffing device 40 is positioned above the transporting and mixing device 30A, 30B so that the cutter blades 50 of the fluffing device 40 may rotate freely without contacting the screws 32A, 32B of the screw means 30A, 30B.

The drive motor 45 and the transmission 44 is supported via a support structure 46 on the first frame structure 60.

One end of the shaft 41 of the fluffing device 40 is connected to the transmission 44 of the drive motor 45.

The ends of the shaft 41 of the fluffing device 40 may be supported via bearings 43A, 43B on the first frame structure 60. This support is not for clarity reasons shown in the figures.

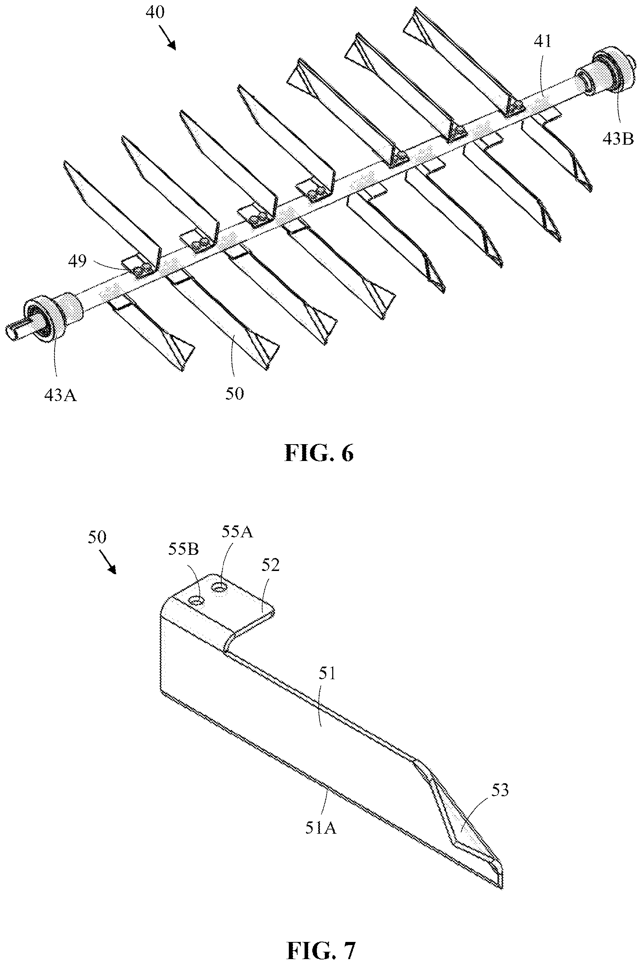

Figure 6 shows an axonometric view of the fluffing device and figure 7 shows an axonometric view of a blade in the fluffing device.

The fluffing device 40 comprises a shaft 41 which may be supported via bearings 43A, 43B on the first frame structure 60. Cutter blades 50 may be attached to the shaft 41 so that the cutter blades 42 protrude radially outwards from the shaft 41. The cutter blades 50 may be attached with bolts 49 to the shaft 41 of the fluffing device 40. The shaft 41 of the fluffing device 40 may be provided with threaded openings receiving the thread of the bolts 49. The other possibility is that the shaft 41 of the fluffing device 40 is provided with holes passing through the shaft 41. The bolts 49 are in such case tightened with nuts threaded on the end of the bolts 49 on the opposite side of the shaft 41 of the fluffing device 40.

The cutter blades 50 may comprise a main blade portion 51 having a substantially rectangular form, a fastening portion 52 extending substantially perpendicularly from the main blade portion 51 at an inner end of the main blade portion 51, and an inclined portion 53 at an outer end of the main blade portion 51. The inclined portion 53 may be made by bending an upper corner of the main blade portion 51 at the outer end of the main blade portion 51. The lower edge 51A of the main blade portion 51 may be sharp in order to be able to effectively cut the material to be composted.

The function of the straight portion of the main portion 51 of the cutter blade 50 is to cut the material to be composted.

The function of the inclined portion 53 of the main portion 51 of the cutter blade 50 is to throw a portion of the material to be composted backwards against the transport direction L1.

The driving motor 45 and the transmission 44 driving the shaft 41 of the fluffing device 40 is positioned outside the container 10. At least the end of the shaft 41 of the fluffing device 40 to which the transmission 44 is connected passes thus trough an opening in the wall of the container 10 to the outside of the container 10. The shaft 41 of the fluffing device 40 may be sealed with a sealing in said opening of the container 10.

The figures show an embodiment in which the transporting and mixing device 30 is formed of two screw means 30A, 30B. This is an advantageous embodiment, but the invention is not restricted to this embodiment. The transporting and mixing device 30 could be formed of any number of screw means 30A, 30B. The transporting and mixing device 30 may thus comprise one screw means, two screw means, three screw means, four screw means etc. The transporting and mixing device 30 may on the other hand comprise at least one screw means, at least two screw means, at least three screw means, at least four screw means etc.

The volume of the container 10 may be adapted according to the need. The volume of the container 10 may e.g. be in the range of 1 to 100 m³. The volume of the container 10 may in an embodiment be in the range of 5 to 6 m³ and the weight of the material to be composted in one batch may be in the range of 1500 to 2000 kg. The container 10 may be filled with material to be composted approximately to one third of the volume of the container 10. There should be enough of air space within the container 10 in order for the composting process to proceed properly. This air space may be provided in the upper portion of the container 10 above the organic material.

The angle α of deflection of the container 10 may be in the range of 10 to 50 degrees, preferably substantially 30 degrees.

The figures show an embodiment in which fresh air F1 is directed into the container 10 from the first air opening 23 positioned in the vicinity of the beginning of the hemispherical wall at the second end 12 of the container 10 and air F2 is directed out from the container 10 from the second air opening 24 positioned in the vicinity of the first end 11 of the container 10. This is an advantageous solution as the fresh air will be blown directly against the portion of organic material that is thrown backwards by the fluffing device 40. It is, however, possible to change the flow direction of the air flow so that air is directed in from the second air opening 24 and out from the first air opening 23 in the container 10. The invention and its embodiments are not limited to the examples described above but may vary within the scope of the claims.

## Claims

1. An apparatus for composting organic material comprising
a container (10) having a first end (11), a second opposite end (12), and a jacket (13) extending between the first end (11) and the second end (12), the second end (12) being formed as a hemispherical wall, the container (10) comprising at least one inlet opening (21) for the organic material and at least one outlet opening (22) for composted material, the container (10) operating in an inclined (α1) position in which the second end (12) of the container (10) is on a higher horizontal level than the first end (11) of the container (10),
a transporting and mixing device (30) positioned in the container (10) for transporting organic material in a transport direction (L1) from the first end (11) to the second end (12) of the container (10) and simultaneously mixing the organic material, the transporting and mixing device (30) directing the organic material towards the hemispheric wall, whereby the organic material is first directed upwards along the hemispheric wall and then turned back against the transport direction (L1),
**characterized in that**
a fluffing device (40) is positioned in the container (10) for cutting the organic material into a smaller particle size and for throwing at least a portion of the organic material backwards towards the first end (11) of the container (10), the fluffing device (40) comprising a shaft (41) extending substantially perpendicularly in relation to the transport direction (L1) and cutter blades (50) extending outwards from the shaft (41).

2. The apparatus according to claim 1, wherein the shaft (41) of the fluffing device (40) is positioned at a first distance (B1) from the beginning of the hemispherical wall at the second end (12) of the container (10), said first distance (B1) being in the range of 0 to 20% of a first length (L10) extending from the beginning of the hemispherical wall at the second end (12) of the container (10) to the first end (11) of the container (10).

3. The apparatus according to claim 1 or 2, wherein the shaft (41) of the fluffing device (40) is positioned substantially at a beginning of the hemispherical wall at the second end (12) of the container (10).

4. The apparatus according to any one of claims 1 to 3, wherein the cutter blades (50) comprises an inclined portion (53) at a radially outer end of the cutter blades (50), said inclined portion (53) throwing at least a portion of the material to be composted backwards towards the first end (11) of the container (10).

5. The apparatus according to any one of claims 1 to 4, wherein the fluffing device (40) is positioned above the transporting and mixing device (30).

6. The apparatus according to any one of claims 1 to 5, wherein the transporting and mixing device (30) is formed of at least two screw means (30A, 30B), each of the screw means (30A, 30B) comprising a shaft (31A, 31B) and a screw (32A, 32B) protruding outwards from a perimeter of the shaft (31A, 31B).

7. The apparatus according to any one of claims 1 to 6, wherein the container (10) is adjustable between a horizontal position (P1) and an inclined (α1) position (P2) in which the second end (12) of the container (10) is on a higher level than the first end (11) of the container (10).

8. The apparatus according to claim 7, wherein the container (10) is pivotably (J1) supported on a second frame structure (70) so that the container (10) is turnable around the pivot point (J1) between the horizontal position (P1) and the inclined (α1) position (P2).

9. The apparatus according to any one of claims 1 to 8, wherein the jacket (13) of the container (10) comprises a first air opening (23) through which air can be directed into the interior of the container (10) and a second air opening (24) through which air can be directed out from the interior of the container (10).

10. The apparatus according to claim 9, wherein the first air opening (23) is positioned substantially above the fluffing device (40).

11. The apparatus according to claim 9, wherein the first air opening (23) is positioned at a second distance (B2) from the beginning of the hemispherical wall at the second end (12) of the container (10), said second distance (B2) being in the range of 0 to 20% of a first length (L10) extending from the beginning of the hemispherical wall at the second end (12) of the container (10) to the first end (11) of the container (10).

12. The apparatus according to any one of claims 9 to 11, wherein the second air opening (24) is positioned at a third distance (B3) from the first end (11) of the container (10) above the transporting and mixing device (30), said third distance (B3) being in the range of 0 to 20% of a first length (L10) extending from the beginning of the hemispherical wall at the second end (12) of the container (10) to the first end (11) of the container (10).

13. A method for composting organic material comprising
providing organic material from at least one inlet opening (21) to a container (10) having a first end (11) and an opposite second end (12) formed as a hemispheric wall, the container (10) operating in an inclined (α1) position in which the second end (12) of the container (10) is on a higher horizontal level than a first end (11) of the container (10),
providing additional material for regulating the oxygen content and/or bacteria and/or enzymes to the container (10) in order to agitate the composting process in the container (10),
transporting the organic material with a transporting and mixing device (30) in a transport direction (L1) from the first end (11) towards the hemispheric wall at the second end (12) of the container (10), whereby the organic material is first directed upwards along the hemispheric wall and then turned back towards the first end (11) of the container (10), the organic material also being mixed during the transport,
removing the composted material from at least one outlet opening (22) in the container (10) after the composting process within the container (10) has been completed,
**characterized by**
cutting the organic material into a smaller particle size and throwing at least a portion of the organic material backwards towards the first end (11) of the container (10) with a fluffing device (40) comprising a shaft (41) extending substantially perpendicularly in relation to the transport direction (L1) and cutter blades (50) extending outwards from the shaft (41).

## Patentansprüche

1. Einrichtung zum Kompostieren von organischem Material, die Folgendes umfasst
einen Behälter (10) mit einem ersten Ende (11), einem zweiten gegenüberliegenden Ende (12) und einem Mantel (13), der sich zwischen dem ersten Ende (11) und dem zweiten Ende (12) erstreckt, wobei das zweite Ende (12) als eine halbkugelförmige Wand gebildet ist, wobei der Behälter (10) mindestens eine Einlassöffnung (21) für das organische Material und mindestens eine Auslassöffnung (22) für kompostiertes Material umfasst, wobei der Behälter (10) in einer geneigten (α1) Position betrieben wird, in der sich das zweite Ende (12) des Behälters (10) auf einer höheren horizontalen Ebene befindet als das erste Ende (11) des Behälters (10),
eine Transport- und Mischvorrichtung (30), die zum Transportieren von organischem Material in eine Transportrichtung (L1) vom ersten Ende (11) zum zweiten Ende (12) des Behälters (10) und gleichzeitigen Mischen des organischen Materials im Behälter (10) positioniert ist, wobei die Transport- und Mischvorrichtung (30) das organische Material zur halbkugelförmigen Wand leitet, wodurch das organische Material zuerst nach oben entlang der halbkugelförmigen Wand geleitet und dann gegen die Transportrichtung (L1) zurückgedreht wird,
**dadurch gekennzeichnet, dass**
eine Aufschüttelvorrichtung (40) zum Schneiden des organischen Materials in eine kleinere Partikelgröße und zum Zurückwerfen mindestens eines Abschnitts des organischen Materials zum ersten Ende (11) des Behälters (10) im Behälter (10) positioniert ist, wobei die Aufschüttelvorrichtung (40) eine Welle (41), die sich mit Bezug auf die Transportrichtung (L1) im Wesentlichen senkrecht erstreckt, und Schneidklingen (50), die sich von der Welle (41) nach außen erstrecken, umfasst.

2. Einrichtung nach Anspruch 1, wobei die Welle (41) der Aufschüttelvorrichtung (40) vom Anfang der halbkugelförmigen Wand am zweiten Ende (12) des Behälters (10) in einem ersten Abstand (B1) positioniert ist, wobei der erste Abstand (B1) im Bereich von 0 bis 20 % einer ersten Länge (L10) liegt, die sich vom Anfang der halbkugelförmigen Wand am zweiten Ende (12) des Behälters (10) zum ersten Ende (11) des Behälters (10) erstreckt.

3. Einrichtung nach Anspruch 1 oder 2, wobei die Welle (41) der Aufschüttelvorrichtung (40) im Wesentlichen an einem Anfang der halbkugelförmigen Wand am zweiten Ende (12) des Behälters (10) positioniert ist.

4. Einrichtung nach einem der Ansprüche 1 bis 3, wobei die Schneidklingen (50) an einem radial äußeren Ende der Schneidklingen (50) einen geneigten Abschnitt (53) umfasst, wobei der geneigte Abschnitt (53) mindestens einen Abschnitt des zu kompostierenden Materials zurück zum ersten Ende (11) des Behälters (10) wirft.

5. Einrichtung nach einem der Ansprüche 1 bis 4, wobei die Aufschüttelvorrichtung (40) über der Transport- und Mischvorrichtung (30) positioniert ist.

6. Einrichtung nach einem der Ansprüche 1 bis 5, wobei die Transport- und Mischvorrichtung (30) aus mindestens zwei Schneckenmitteln (30A, 30B) gebildet ist wobei jedes der Schneckenmittel (30A, 30B) eine Welle (31A, 31B) und eine Schnecke (32A, 32B), die von einem Umfang der Welle (31A, 31B) nach außen vorsteht, umfasst.

7. Einrichtung nach einem der Ansprüche 1 bis 6, wobei der Behälter (10) zwischen einer horizontalen Position (P1) und einer geneigten (α1) Position (P2), in der sich das zweite Ende (12) des Behälters (10) auf einer höheren Ebene befindet als das erste Ende (11) des Behälters (10), einstellbar ist.

8. Einrichtung nach Anspruch 7, wobei der Behälter (10) auf einer zweiten Rahmenstruktur (70) derart schwenkbar (J1) gestützt wird, dass der Behälter (10) um den Schwenkpunkt (J1) zwischen der horizontalen Position (P1) und der geneigten (α1) Position (P2) drehbar ist.

9. Einrichtung nach einem der Ansprüche 1 bis 8, wobei der Mantel (13) des Behälters (10) eine erste Luftöffnung (23), durch die Luft in das Innere des Behälters (10) geleitet werden kann, und eine zweite Luftöffnung (24), durch die Luft vom Inneren des Behälters (10) herausgeleitet werden kann, umfasst.

10. Einrichtung nach Anspruch 9, wobei die erste Luftöffnung (23) im Wesentlichen über der Aufschüttelvorrichtung (40) positioniert ist.

11. Einrichtung nach Anspruch 9, wobei die erste Luftöffnung (23) vom Anfang der halbkugelförmigen Wand am zweiten Ende (12) des Behälters (10) in einem zweiten Abstand (B2) positioniert ist, wobei der zweite Abstand (B2) im Bereich von 0 bis 20 % einer ersten Länge (L10) liegt, die sich vom Anfang der halbkugelförmigen Wand am zweiten Ende (12) des Behälters (10) zum ersten Ende (11) des Behälters (10) erstreckt.

12. Einrichtung nach einem der Ansprüche 9 bis 11, wobei die zweite Luftöffnung (24) vom ersten Ende (11) des Behälters (10) über der Transport- und Mischvorrichtung (30) in einem dritten Abstand (B3) positioniert ist, wobei der dritte Abstand (B3) im Bereich von 0 bis 20 % einer ersten Länge (L10) liegt, die sich vom Anfang der halbkugelförmigen Wand am zweiten Ende (12) des Behälters (10) zum ersten Ende (11) des Behälters (10) erstreckt.

13. Verfahren zum Kompostieren von organischem Material, das Folgendes umfasst
Bereitstellen von organischem Material von mindestens einer Einlassöffnung (21) zu einem Behälter (10) mit einem ersten Ende (11) und einem gegenüberliegenden zweiten Ende (12), das als eine halbkugelförmige Wand gebildet ist, wobei der Behälter (10) in einer geneigten (α1) Position betrieben wird, in der sich das zweite Ende (12) des Behälters (10) auf einer höheren horizontalen Ebene befindet als ein erstes Ende (11) des Behälters (10),
Bereitstellen von zusätzlichem Material zum Regeln des Sauerstoffgehalts und/oder der Bakterien und/oder der Enzyme zum Behälter (10), um den Kompostierungsprozess im Behälter (10) durchzurühren,
Transportieren des organischen Materials mit einer Transport- und Mischvorrichtung (30) in eine erste Transportrichtung (L1) vom ersten Ende (11) zur halbkugelförmigen Wand am zweiten Ende (12) des Behälters (10), wodurch das organische Material zuerst entlang der halbkugelförmigen Wand nach oben geleitet und dann zum ersten Ende (11) des Behälters (10) zurückgedreht wird, wobei das organische Material während des Transports auch gemischt wird,
Entfernen des kompostierten Materials aus mindestens einer Auslassöffnung (22) im Behälter (10), nachdem der Kompostierungsprozess im Behälter (10) abgeschlossen ist,
**gekennzeichnet durch**
Schneiden des organischen Materials in eine kleinere Partikelgröße und Zurückwerfen mindestens eines Abschnitts des organischen Materials zum ersten Ende (11) des Behälters (10) mit einer Aufschüttelvorrichtung (40), die eine Welle (41), die sich mit Bezug auf die Transportrichtung (L1) im Wesentlichen senkrecht erstreckt, und Schneidklingen (50), die sich von der Welle (41) nach außen erstrecken, umfasst.

## Revendications

1. Appareil de compostage de matériau organique comprenant :
un contenant (10) ayant une première extrémité (11), une seconde extrémité (12) opposée, et une chemise (13) s'étendant entre la première extrémité (11) et la seconde extrémité (12), la seconde extrémité (12) étant formée comme une paroi hémisphérique, le contenant (10) comprenant au moins une ouverture d'entrée (21) pour le matériau organique et au moins une ouverture de sortie (22) pour le matériau composté, le contenant (10) fonctionnant dans une position inclinée (al) dans laquelle la seconde extrémité (12) du contenant (10) est sur un niveau horizontal plus haut que la première extrémité (11) du contenant (10),
un dispositif de transport et de mélange (30) positionné dans le contenant (10) pour transporter le matériau organique dans une direction de transport (L1) de la première extrémité (11) à la seconde extrémité (12) du contenant (10) et mélanger simultanément le matériau organique, le dispositif de transport et de mélange (30) dirigeant le matériau organique vers la paroi hémisphérique, moyennant quoi le matériau organique est tout d'abord dirigé vers le haut le long de la paroi hémisphérique et ensuite se retourne contre la direction de transport (L1),
**caractérisé en ce que** :
un dispositif de peluchage (40) est positionné dans le contenant (10) pour couper le matériau organique en une plus petite taille particulaire et pour rejeter au moins une partie du matériau organique vers la première extrémité (11) du contenant (10), le dispositif de peluchage (40) comprenant un arbre (41) s'étendant sensiblement perpendiculairement par rapport à la direction de transport (L1) et des lames de coupe (50) s'étendant vers l'extérieur à partir de l'arbre (41).

2. Appareil selon la revendication 1, dans lequel l'arbre (41) du dispositif de peluchage (40) est positionné à une première distance (B1) du commencement de la paroi hémisphérique au niveau de la seconde extrémité (12) du contenant (10), ladite première distance (B1) étant dans une plage de 0 à 20% d'une première longueur (L10) s'étendant depuis le commencement de la paroi hémisphérique au niveau de la seconde extrémité (12) du contenant (10) jusqu'à la première extrémité (11) du contenant (10).

3. Appareil selon la revendication 1 ou 2, dans lequel l'arbre (41) du dispositif de peluchage (40) est positionné sensiblement à un commencement de la paroi hémisphérique au niveau de la seconde extrémité (12) du contenant (10).

4. Appareil selon l'une quelconque des revendications 1 à 3, dans lequel les lames de coupe (50) comprennent une partie inclinée (53) au niveau d'une extrémité radialement externe des lames de coupe (50), ladite partie inclinée (53) rejetant au moins une partie du matériau à composter vers la première extrémité (11) du contenant (10).

5. Appareil selon l'une quelconque des revendications 1 à 4, dans lequel le dispositif de peluchage (40) est positionné au-dessus du dispositif de transport et de mélange (30).

6. Appareil selon l'une quelconque des revendications 1 à 5, dans lequel le dispositif de transport et de mélange (30) est formé avec au moins deux moyens de vis (30A, 30B), chacun des moyens de vis (30A, 30B) comprenant un arbre (31A, 31B) et une vis (32A, 32B) faisant saillie vers l'extérieur à partir d'un périmètre de l'arbre (31A, 31B).

7. Appareil selon l'une quelconque des revendications 1 à 6, dans lequel le contenant (10) est ajustable entre une position horizontale (P1) et une position inclinée (α1) (P2) dans laquelle la seconde extrémité (12) du contenant (10) est sur un niveau plus élevé que la première extrémité (11) du contenant (10).

8. Appareil selon la revendication 7, dans lequel le contenant (10) est supporté de manière pivotante (J1) sur une seconde structure de bâti (70), de sorte que le contenant (10) peut tourner autour du point de pivot (J1) entre la position horizontale (P1) et la position inclinée (α1) (P2).

9. Appareil selon l'une quelconque des revendications 1 à 8, dans lequel la chemise (13) du contenant (10) comprenant une première ouverture d'air (23) à travers laquelle l'air peut être dirigé dans l'intérieur du contenant (10) et une seconde ouverture d'air (24) à travers laquelle l'air peut être dirigé hors de l'intérieur du contenant (10).

10. Appareil selon la revendication 9, dans lequel la première ouverture d'air (23) est positionnée sensiblement au-dessus du dispositif de peluchage (40).

11. Appareil selon la revendication 9, dans lequel la première ouverture d'air (23) est positionnée à une deuxième distance (B2) du commencement de la paroi hémisphérique au niveau de la seconde extrémité (12) du contenant (10), ladite deuxième distance (B2) étant dans la plage de 0 à 20% d'une première longueur (L10) s'étendant à partir du commencement de la paroi hémisphérique au niveau de la seconde extrémité (12) du contenant (10) jusqu'à la première extrémité (11) du contenant (10).

12. Appareil selon l'une quelconque des revendications 9 à 11, dans lequel la seconde ouverture d'air (24) est positionnée à une troisième distance (B3) de la première extrémité (11) du contenant (10) au-dessus du dispositif de transport et de mélange (30), ladite troisième distance (B3) étant dans la plage de 0 à 20% d'une première longueur (L10) s'étendant à partir du commencement de la paroi hémisphérique au niveau de la seconde extrémité (12) du contenant (10) jusqu'à la première extrémité (11) du contenant (10).

13. Procédé pour composter un matériau organique comprenant les étapes consistant à :
prévoir un matériau organique à partir d'au moins une ouverture d'entrée (21) jusqu'à un contenant (10) ayant une première extrémité (11) et une seconde extrémité (12) opposée formée comme une paroi hémisphérique, le contenant (10) fonctionnant dans une position inclinée (al) dans laquelle la seconde extrémité (12) du contenant (10) est sur un niveau horizontal plus haut qu'une première extrémité (11) du contenant (10),
prévoir le matériau supplémentaire pour réguler la teneur en oxygène et/ou en bactéries et/ou en enzymes dans le contenant (10) afin d'agiter le processus de compostage dans le contenant (10),
transporter le matériau organique avec un dispositif de transport et de mélange (30) dans une direction de transport (L1) de la première extrémité (11) vers la paroi hémisphérique au niveau de la seconde extrémité (12) du contenant (10), moyennant quoi le matériau organique est tout d'abord dirigé vers le haut le long de la paroi hémisphérique et ensuite se retourne vers la première extrémité (11) du contenant (10), le matériau organique étant également mélangé pendant le transport,
retirer le matériau composté par au moins une ouverture de sortie (22) dans le contenant (10) après l'achèvement du processus de compostage à l'intérieur du contenant (10),
**caractérisé par** l'étape consistant à :
couper le matériau organique en une plus petite taille particulaire et rejeter au moins une partie du matériau organique vers la première extrémité (11) du contenant (10) avec un dispositif de peluchage (40) comprenant un arbre (41) s'étendant sensiblement perpendiculairement par rapport à la direction de transport (L1) et des lames de coupe (50) s'étendant vers l'extérieur à partir de l'arbre (41).
